# EUROPEAN PATENT APPLICATION

(11) **EP 4 606 814 A1**
(43) Date of publication of application: **27.08.2025**
(21) Application number: 23879148.7
(22) Date of filing: 18.10.2023
(51) Int. Cl.: C07K 5/062, C07D 471/10, A61K 31/438

(54) **CRYSTAL FORM AND AMORPHOUS SUBSTANCE OF INDOLINE SPIRO COMPOUND, AND PREPARATION METHOD THEREFOR AND USE THEREOF**

(30) Priority: 21.10.2022 CN 202211297102; 09.10.2023 CN 202311300559
(71) Applicant: Changchun Genescience Pharmaceutical Co., Ltd., Changchun, Jilin 130012 (CN)
(72) Inventor: LIN, Daizong, Changchun, Jilin 130012 (CN); HUANG, Qiang, Changchun, Jilin 130012 (CN); SHAO, Wenbo, Changchun, Jilin 130012 (CN); SUN, Yi, Changchun, Jilin 130012 (CN); LOU, Xiyu, Changchun, Jilin 130012 (CN); LIU, Qingyin, Changchun, Jilin 130012 (CN); XU, Xueyu, Changchun, Jilin 130012 (CN); YANG, Fanglong, Changchun, Jilin 130012 (CN); WANG, Siqin, Changchun, Jilin 130012 (CN); JIN, Lei, Changchun, Jilin 130012 (CN)
(74) Representative: Gille Hrabal Partnerschaftsgesellschaft mbB Patentanwälte
(86) International application number: PCT/CN2023/125251
(87) International publication number: WO 2024/083160

(57) **Abstract**

Provided are a crystal form and an amorphous substance of an indoline spiro compound represented by formula (1), and a preparation method therefor and the use thereof. The X-ray powder diffraction pattern of the crystal form comprises characteristic peaks at 5.43±0.20°, 12.28±0.20° and 17.91±0.20° 2θ, as determined by using Cu-Kα radiation; and the XRPD spectrum of the amorphous substance has no obvious diffraction peak. The provided crystal form and the amorphous substance have high purity and good stability, and are beneficial to drug formation.

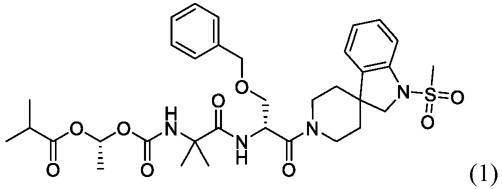

## Description

The present application claims priority to Chinese Prior Patent Application 202211297102.5, entitled "CRYSTAL FORM AND AMORPHOUS SUBSTANCE OF INDOLINE SPIRO COMPOUND, AND PREPARATION METHOD THEREFOR AND USE THEREOF" filed with the Chinese Patent Office on October 21, 2022 and Chinese Prior Patent Application 202311300559.1, entitled "CRYSTAL FORM AND AMORPHOUS SUBSTANCE OF INDOLINE SPIRO COMPOUND, AND PREPARATION METHOD THEREFOR AND USE THEREOF" filed with the Chinese Patent Office on October 9, 2023, which are incorporated by reference in the present application.

### TECHNICAL FIELD

The present invention belongs to the field of pharmaceutical compounds, and particularly relates to a crystal form and an amorphous substance of an indoline spiro compound, and a preparation method therefor and use thereof.

### BACKGROUND

Human Growth Hormone (GH) is a kind of peptide hormone secreted by anterior pituitary, it is formed by 191 amino acids and directly or indirectly acts on peripheral organs by inducing the synthesis of an insulin-like growth factor 1 (IGF-1) or an epidermal growth factor (EGF), and its main physiological functions comprise promoting linear growth of the body, promoting cell proliferation of muscle and skin, and it plays an important role in regeneration of tissues after trauma.

Ghrelin is a kind of endogenous growth hormone releasing peptide containing 28 amino acids, and it is an endogenous ligand for a Growth Hormone Secretagogue Receptor 1a (GHSR 1a). It has been confirmed in in-vivo and in-vitro experiments that Ghrelin can significantly promote the secretion of growth hormone. It was also found in clinical studies that intravenous Ghrelin can strongly stimulate the release of growth hormone in a dose-dependent manner.

It is considered that the released GH can treat physiological or pathophysiological disorders characterized by defects in the secretion of growth hormone, and treat disorders alleviated by the anabolic effects of growth hormone. In clinical findings, GH are promising for treating disorders such as muscle mass loss, adipose tissue aggregation, bone demineralization, and reduced tissue regeneration capacity after injury.

GH is synthesized and stored in pituitary gland, but is controlled by hypothalamic hormones to release. At present, there are two hormones involved in the release of GH: growth hormone-releasing hormone (GHRH) and inhibitory hormone growth hormone release inhibiting hormone (SRIF). Mostly, GH defects involve the release of GH (hypothalamic defect) rather than the synthesis of GH (pituitary defect). Therefore, using GHSR agonists for stimulating the release of GH in pituitary may be a new therapy to replace recombinant human growth hormone.

GHSR has two subtypes, namely, 1a and 1b, in which, the subtype 1a is a functional receptor subtype, and the function of subtype 1b needs to be further studied. In central nervous system, GHSR 1a is distributed in hypothalamus and a plurality of regions outside hypothalamus, including pituitary, hypothalamic arcuate nucleus, and ventromedial nucleus. Peripherally, GHSR also has low expression in thyroid, pancreas, myocardium, etc. Therefore, Ghrelin and the receptor GHSR 1a thereof may be involved in regulation of a plurality of functions in the body.

It was found in studies that some peptide or peptidomimetic clinical compounds show GHSR agonistic activity and have the effect of inducing the release of GH. The compounds currently entering clinical study comprise examorelin, tabimorelin, pralmorelin, ibutamoren, tesamorelin, anamorelin and macimorelin, among which, the injectable polypeptide tesamorelin (used for reducing excessive abdominal fat in HIV-infected patients) and small molecule peptidomimetic macimorelin have been approved by FDA. Macimorelin is the only drug that is approved for being taken orally for diagnosing adult growth hormone deficiency, but it also has disadvantages such as poor oral bioavailability and risk of potential cardiotoxicity.

It was also found in related studies that in addition to mediating and inducing GH secretion by GHSR 1a activation, the GHSR agonists can also mediate to produce other physiological functions through different receptors of other GHS receptor families, or different binding sites on GHSR (such as GHSR 1b, a gastrointestinal motilin receptor 1a, a neurotensin receptor and a TRH receptor). Therefore, the use of the GHSR agonists in the field of gastrointestinal indications was newly developed, and there are currently no drugs on the market for this indication, among which, ulimorelin and relamorelin have entered phase III clinical studies.

It has been confirmed that Ghrelin can play a prokinetic effect on gastrointestinal peristalsis through vagus nerve and pelvic nerves, but the half life of Ghrelin is short, which blocks its drug formation performance. It is needed to develop GHSR agonists with enhanced pharmacokinetics to alleviate gastrointestinal impairment in animals and humans.

In order to overcome the above-mentioned technical problems, the applicant independently developed and obtained a compound with a completely new molecular structure, its structural formula is: 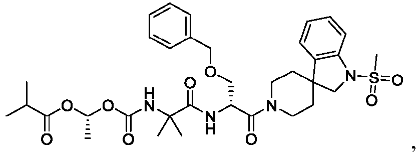 and its chemical name is: (4R,11R)-7,7-dimethyl-4-(1-(methylsulfonyl)spiro[indoldoline-3,4'-piperidine]-1'-carbonyl)-6,9-dioxo-1-phenyl-2,10-dioxa-5,8-diazadodecane-11-ylisobutyrate, and relevant contents are recorded in the patent application PCT/CN2022/088656. Pharmacodynamic experiments showed that the compound has a good clinical use prospect and can be used for diagnosing, preventing and/or treating growth hormone-dependent diseases or disorders; preferably, the diseases or disorders involve growth hormone deficiency or growth hormone dependence, for example, the diagnosis of patients with growth hormone deficiency, slow growth and short stature of children with growth hormone deficiency, and treatment of other diseases that can be alleviated by physiological effects of growth hormone comprise but are not limited to: energy balance and regulation of food intake; treatment of lipogenesis, obesity and weight loss; treatment of cachexia; improvement of gastrointestinal motility, treatment of gastroparesis and diabetic gastroparesis, and postoperative intestinal obstruction; increase in muscle mass and skin thickness, decrease in fatty substances, and slight increase in bone density in elderly patient population; and treatment of burns, AIDS and cancer conditions, as well as healing of wounds and bones.

Moreover, studying the compound to fit a solid form of a formed drug, such as solid forms that improve stability, hygroscopicity and/or efficacy, so as to achieve good effects in pharmaceutical and drug use stages, is a technical problem that is urgent to be solved.

### SUMMARY

All contents involved in the patent application PCT/CN2022/088656 are added to the present invention by citation.

In order to ameliorate the above-mentioned technical problem, the present invention provides a crystal form of a compound represented by Formula (1), 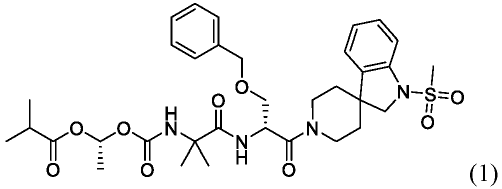 an X-ray powder diffraction pattern of the crystal form comprises characteristic peaks at 5.43±0.20°, 12.28±0.20° and 17.91±0.20° 2θ, as determined by using Cu-Kα radiation.

According to the embodiments of the present invention, the X-ray powder diffraction pattern of the crystal form comprises characteristic peaks at 5.43±0.20°, 12.28±0.20°, 17.91±0.20°, 18.54±0.20°, 18.87±0.20°, 20.54±0.20° and 21.69±0.20° 2θ, as determined by using Cu-Kα radiation.

According to the embodiments of the present invention, the X-ray powder diffraction pattern of the crystal form comprises characteristic peaks at 5.43±0.20°, 7.96±0.20°, 9.72±0.20°, 12.28±0.20°, 13.16±0.20°, 17.69±0.20°, 17.91±0.20°, 18.16±0.20°, 18.54±0.20°, 18.87±0.20°, 19.51±0.20°, 20.31±0.20°, 20.54±0.20°, 21.69±0.20° and 21.86±0.20° 2θ, as determined by using Cu-Kα radiation.

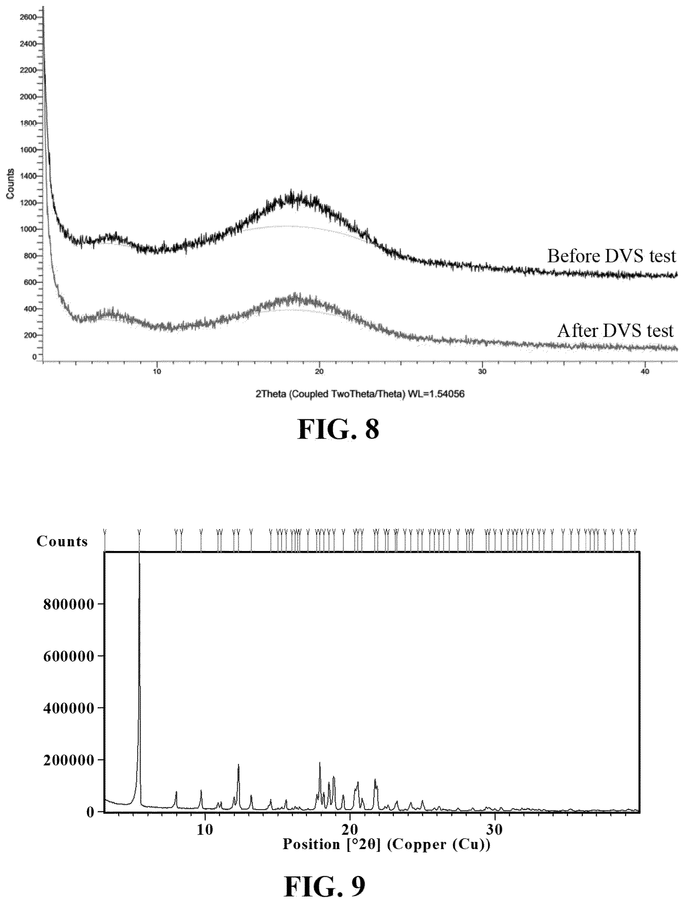

According to the embodiments of the present invention, the crystal form has an XRPD spectrum substantially represented by FIG. 9.

According to the embodiments of the present invention, the crystal form is an anhydrous substance.

According to the embodiments of the present invention, the crystal form has a weight loss not greater than 3wt% before temperature of 150°C, such as not greater than 2wt%.

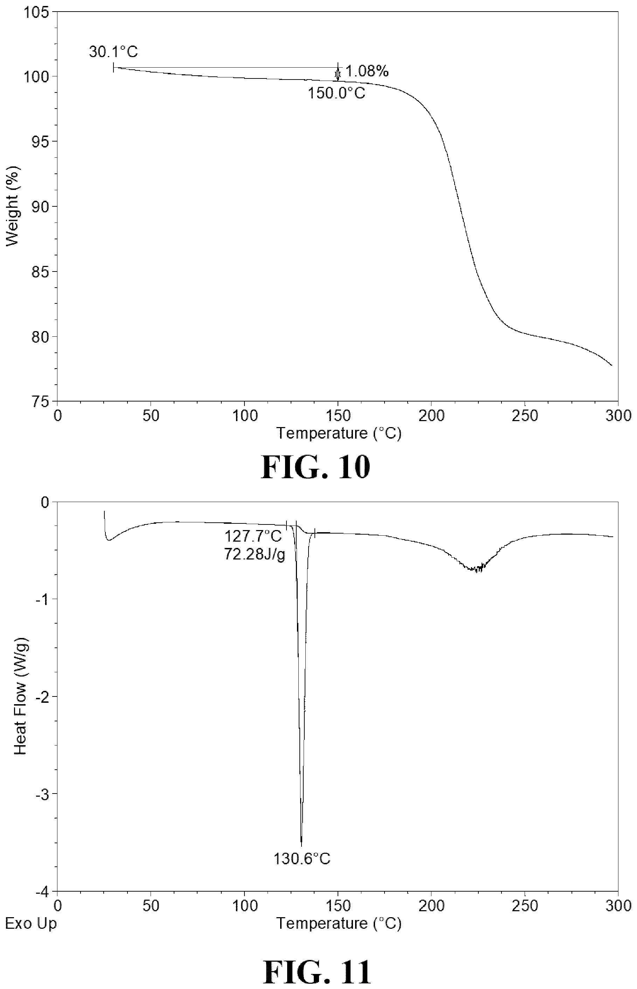

According to the embodiments of the present invention, the crystal form has a TGA pattern substantially represented by FIG. 10.

According to the embodiments of the present invention, the crystal form has a sharp endothermic peak at a peak temperature of 130.6±2°C.

According to the embodiments of the present invention, the crystal form has a DSC pattern substantially represented by FIG. 11.

The present invention further provides a preparation method for the crystal form, and the preparation method comprises the following steps: dissolving the compound represented by Formula (1) in a solvent A, and cooling to precipitate a solid so as to obtain the crystal form; and
the solvent A can be selected from a group consisting of methanol, ethanol, isopropanol, tert-butanol, n-butanol, acetone, tetrahydrofuran, methyltetrahydrofuran, ethyl formate, ethyl acetate, isopropyl acetate, n-hexane, n-heptane, cyclohexane, methyl tert-butyl ether, toluene, dichloromethane, chloroform, DMSO, water, acetonitrile, isopropyl ether and the like, such as a mixed solvent selected from ethanol/water, DMSO/water, acetone/water, ethanol/n-hexane, ethanol/cyclohexane, acetonitrile/n-heptane, ethanol/n-heptane, isopropanol/n-heptane, methanol/n-heptane, methanol/n-hexane, and acetone/n-heptane and combinations thereof.

According to the embodiments of the present invention, the dissolving process is performed in a heating condition.

According to the embodiments of the present invention, the cooling process is to decrease the temperature to room temperature.

According to the embodiments of the present invention, the cooling process is a slow cooling process.

According to the embodiments of the present invention, the method further comprises posttreatment on the solid, such as filtration and/or washing.

The present invention also provides an amorphous substance of the compound represented by Formula (1), and the XRPD spectrum of the amorphous substance does not have obvious diffraction peak.

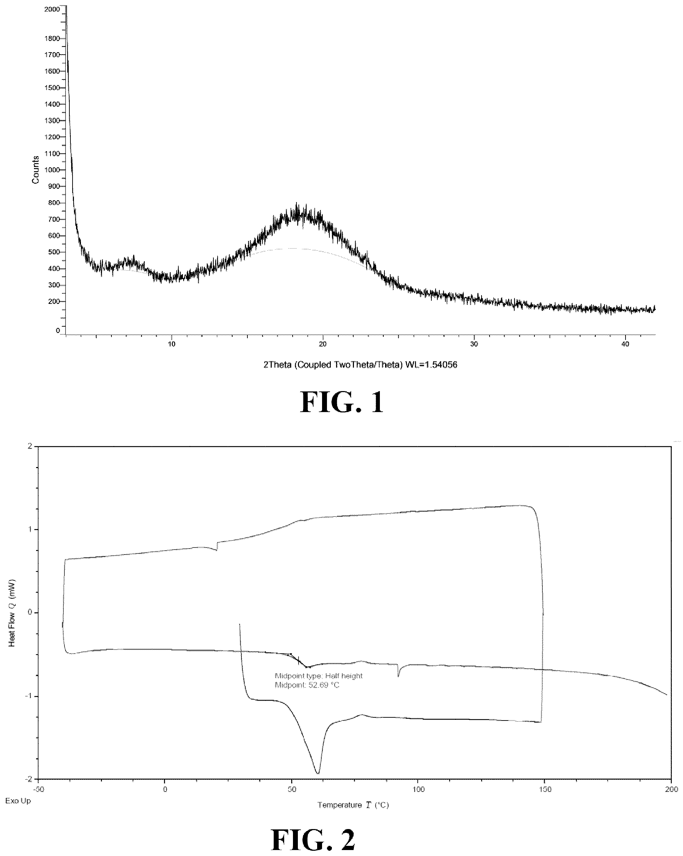

According to the embodiments of the present invention, the amorphous substance has an XRPD spectrum substantially represented by FIG. 1.

The present invention further provides a preparation method of the amorphous substance, and the preparation method comprises the following steps: dissolving the compound represented by Formula (1) in a solvent B, adding the obtained clear solution to a solvent C, and stirring to precipitate a solid so as to obtain the amorphous substance; and
the solvent B is a good solvent for the compound represented by Formula (1), and can be, for example, selected from a group consisting of DMF, DMA, NMP, acetonitrile, THF, DMSO, methyl tert-butyl ether, isopropyl ether, methanol, ethanol, isopropanol, acetone and combinations of two or more solvents thereof, and the like; and
the solvent C is a poor solvent for the compound represented by Formula (1), and can be, for example, selected from a group consisting of water, n-heptane, n-hexane and cyclohexane and combinations of two or more solvents thereof.

According to the embodiments of the present invention, the clear solution is dropwise added to the solvent C.

According to the embodiments of the present invention, the method further comprises posttreatment on the solid, such as filtration and/or washing.

The present invention further provides a pharmaceutical composition, which contains the crystal form and/or the amorphous substance.

According to the embodiments of the present invention, the pharmaceutical composition further contains pharmaceutically acceptable excipients, including, but are not limited to, for example, one or more than two of vehicles, fillers, lubricants, adhesives, disintegrants, inorganic salts, solvents, dissolution aids, suspensions, isotonic agents, buffer solutions, preservatives, antioxidants, colorants, foaming agents, flavoring agents and the like.

According to the embodiments of the present invention, the pharmaceutical composition further comprises a second active ingredient, besides the crystal form and/or the amorphous substance, for example, the second active ingredient is a growth development-related drug, such as a GHSR agonist or growth hormone.

In some embodiments, the crystal form and/or amorphous substance and the second active ingredient can be separately applied or co-applied during treatment.

The present invention further provides use of the crystal form and/or amorphous substance and/or pharmaceutical composition for the manufacture of a medicament for diagnosing, preventing and/or treating growth hormone deficiency or growth hormone-dependent diseases (or disorders).

According to the embodiments of the present invention, the diagnosis of the diseases such as patients with growth hormone deficiency, slow growth and short stature of children with growth hormone deficiency, and treatment of other diseases that can be alleviated by physiological effects of growth hormone comprise but are not limited to: energy balance and regulation of food intake; treatment of lipogenesis, obesity and weight loss; treatment of cachexia; improvement of gastrointestinal motility, treatment of gastroparesis and diabetic gastroparesis, and postoperative intestinal obstruction; increase in muscle mass and skin thickness, decrease in fatty substances, and slight increase in bone density in elderly patient population; and treatment of burns, AIDS and cancer conditions, as well as healing of wounds and bones.
In some embodiments, the pharmaceutical composition is a pharmaceutical formulation, for example, a GHSR agonist.

The present invention further provides a pharmaceutical formulation, which contains the crystal form and/or the amorphous substance, or is prepared from the pharmaceutical composition.

According to the embodiments of the present invention, the preparation can be powder, tablets (such as coated tablets, slow-release or controlled-release tablets), lozenges, capsules (such as soft capsules or hard capsules), granules, pills, dispersible powder, suspensions, solutions, emulsions, elixirs, syrups, aerosols, creams, ointments, gels, injections, freeze-dried powder injections or suppositories, and other forms.

According to the embodiments of the present invention, the pharmaceutical formulation can be administered in any of the following ways: oral administration, buccal administration, sublingual administration, inhaled administration, local application, parenteral intravenous, subcutaneous, acupuncture point or intramuscular injection, and rectal administration.

The present invention further provides a method for diagnosing, preventing and/or treating growth hormone deficiency or growth hormone-dependent diseases (or disorders), and the method comprises: administering a therapeutically effective amount of the crystal form and/or amorphous substance or pharmaceutical composition to patients.

According to the embodiments of the present invention, the diseases are limited as above.

### Beneficial effects

The present invention provides the crystal form and the amorphous substance of a compound represented by Formula (1), and the preparation method therefor and the use thereof. According to the present invention, the obtained crystal form and amorphous substance of the compound represented by Formula (1) have good stability and solubility, low hygroscopicity, long-term storage and good repeatability, and are suitable for drug development.

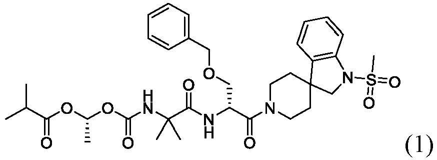

The compound represented by Formula (1) has high crystal purity, good crystal form stability under lighting, high temperature and high humidity conditions, and low hygroscopicity, can be stored for a long term, and is suitable for drug development; and the preparation process of the compound is stable and good in repeatability, and can be suitable for industrial production.

In the present invention, the prepared amorphous substance of the compound represented by Formula (1) has high purity, has good solubility in most solvents, has good physical stability and good chemical stability under lighting, high temperature and high humidity conditions, and is suitable for drug development.

Definitions and descriptions of terms

Unless otherwise specified, the definitions of terms recorded in the description and claims of the present application, including definitions as examples, exemplary definitions, preferred definitions, definitions of specific compounds in embodiments, etc., can be arbitrarily combined and combined with each other. Such combinations and combinations shall fall within the scope of the description of the present application.

The term "therapeutically effective amount" refers to the amount of the crystal form, amorphous substance, second active ingredient sufficient to achieve the intended application (including, but not limited to, the treatment of diseases defined below) in the present invention. The therapeutically effective amount may vary depending on the following factors: intended use (in vitro or in vivo), or a subject and diseases/ disorders being treated, such as the weight and age of the subject, the severity of the diseases/disorders, and the mode of administration, which can be easily determined by those of ordinary skill in the art. The specific dosage will vary depending on the following factors: the selected specific active ingredient, dosing regimen, whether to administrate in combination with other compounds, the timing of dosing, the organization of the drug administered, and the physical delivery system for carrying.

The term "patients" refers to any animal, including mammals, preferably mice, rats, other rodents, rabbits, dogs, cats, pigs, cattle, sheep, horses, or primates, most preferably humans.

The term "130.6±2°C" refers to 128.6-132.6°C, for example, 128.6°C, 129.0°C, 130.0°C, 131.0°C, 132.0°C, 132.6°C or the value of any two of them.

### BRIEF DESCRIPTION OF THE DRAWINGS

FIG. 1 is an XRPD spectrum of an amorphous substance of a compound represented by Formula (1);
FIG. 2 is a DSC pattern of an amorphous substance of a compound represented by Formula (1);
FIG. 3 is a TGA pattern of an amorphous substance of a compound represented by Formula (1);
FIG. 4 is a PLM pattern of an amorphous substance of a compound represented by Formula (1);
FIG. 5 is a SEM pattern of an amorphous substance of a compound represented by Formula (1);
FIG. 6 is a ¹H NMR pattern of an amorphous substance of a compound represented by Formula (1);
FIG. 7 is a DVS pattern of an amorphous substance of a compound represented by Formula (1);
FIG. 8 is an XRPD superposition pattern of an amorphous substance of a compound represented by Formula (1) before and after DVS test;
FIG. 9 is an XRPD spectrum of a crystal form of a compound represented by Formula (1);
FIG. 10 is a TGA pattern of a crystal form of a compound represented by Formula (1);
FIG. 11 is a DSC pattern of a crystal form of a compound represented by Formula (1);
FIG. 12 is a DVS pattern of a crystal form of a compound represented by Formula (1);
FIG. 13 is an XRPD superposition pattern of a crystal form of a compound represented by Formula (1) before and after DVS test;

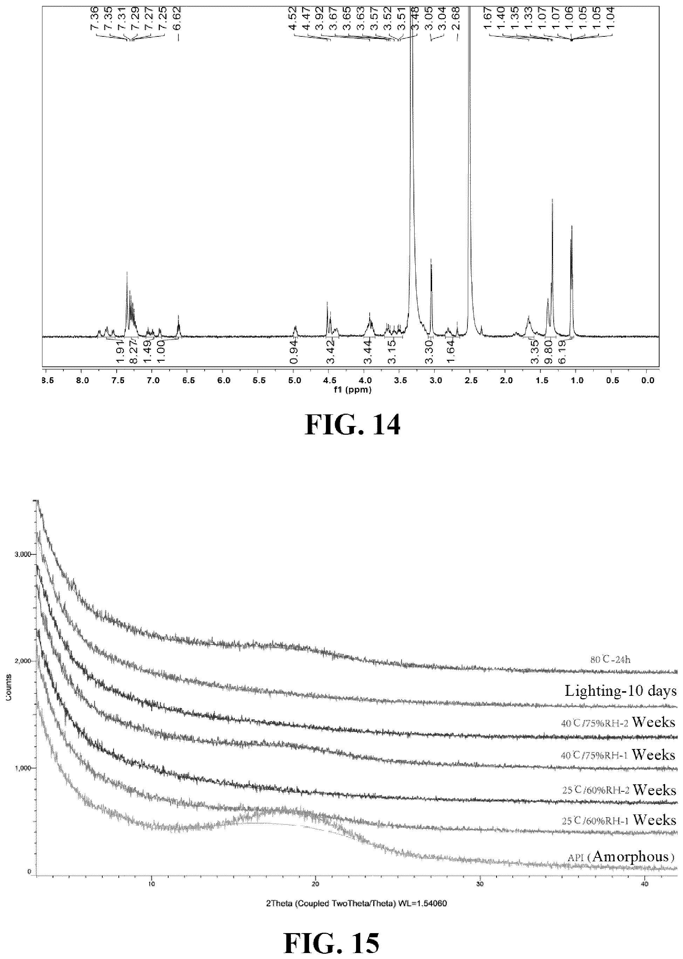

FIG. 14 is a ¹H NMR pattern of a crystal form of a compound represented by Formula (1);
FIG. 15 is an XRPD superposition pattern of solid stability of an amorphous substance of a compound represented by Formula (1); and

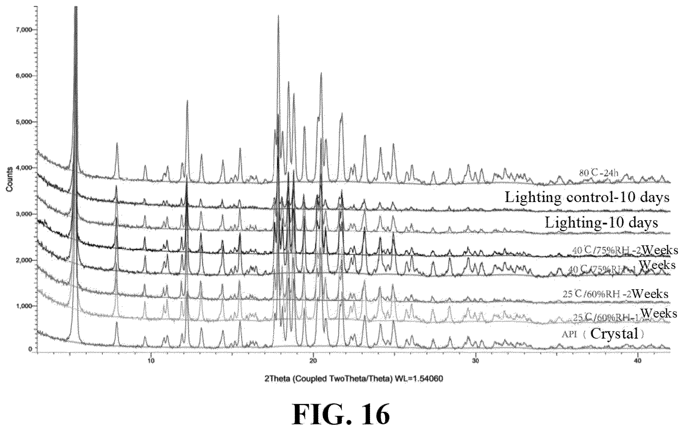

FIG. 16 is an XRPD superposition pattern of stability of a crystal form of a compound represented by Formula (1).

### DETAILED DESCRIPTION

The technical solution of the present invention will be further described in detail in conjunction with specific embodiments below. It is to be understood that the following embodiments are only illustrative and explain the present invention and should not be construed as limiting the scope of protection of the present invention. All techniques realized on the basis of the foregoing of the present invention are covered by the protection scope of the present invention.

Unless otherwise stated, raw materials and reagents used in the following embodiments are commercially available, or may be prepared by known methods.

### Preparation Example Preparation of compound represented by Formula (1)

### (4R,11R)-7,7-dimethyl-4-(1-(methylsulfonyl)spiro[indoldoline-3,4'-piperidine]-1'-carbonyl)-6,9-dioxo-1-phenyl-2,10-dioxa-5,8-diazadodecane-11-ylisobutyrate

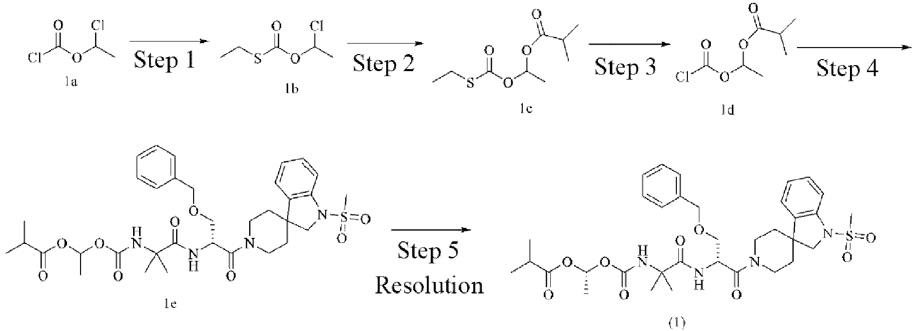

### Step 1 Preparation of o-(1-chloroethyl)thiocarbonate(1b)

1-chloroethyl chloroformate **1a** (3 g, 0.02 mol) was dissolved in dichloromethane (10 mL), and then tetrabutylammonium bromide (TBAB) (0.34 g) was added. To above mixture was added dropwise sodium thioethylate (1.76 g; 0.02 mol) dissolved in water (10mL). The reaction mixture was layered after stirring at 25°C for 16 h. Then the organic layer was washed with water (20 mL), dried over anhydrous sodium sulfate , and then concentrated to give the compound **1b**(2.0 g, yield: 56 %) as yellow oil.

¹H NMR (400 MHz, CDCl₃) δ 6.60 (q, J = 5.8 Hz, 1H), 2.97-2.86 (m, 2H), 1.81 (d, J = 5.8 Hz, 3H), 1.34 (t, J = 7.4 Hz, 3H).

### Step 2 Preparation of 1-{[(ethylthio)carbonyl]oxy}ethyl isobutyrate (1c)

The compound **1b** (700 mg; 4.15 mmol) was dissolved in isobutyric acid (2.2 g, 25 mmol), then N,N-diisopropyelethylamine (1.6 g, 12.5 mmol) was added, and the reaction solution was stirred at 55°C for 48 h. The reaction mixture was quenched with water (20 mL), extracted with ethyl acetate (20 mL), washed with saturated sodium bicarbonate (3x30 mL) and subjected to water washing bybrine (2x20 mL), and then concentrated to give residue **1c** (850 mg, light yellow oil) .

¹HNMR(400MHz,CDCl₃):δ 6.94 (q, J=5.4Hz, 1H), 2.92-2.83 (m, 2H), 2.59(dt, J=4.3, 2.4Hz, 1H), 1.50(d, J=5.5Hz, 3H),1.32(td, J=7.3, 3.6Hz, 3H), 1.20(d, J=7.0Hz, 6H).

### Step 3 Preparation of ethyl 1-((chlorocarbonyl)oxyisobutyrate (1d)

Sulfonyl chloride (147 mg, 1.09 mmol) was slowly dropwise added to the compound **1c** (200 mg, 0.91 mmol) at 0-5°C, the reaction mixture was stirred at 25°C for 45 min. The reaction mixture was concentrated to give residue **1d**, which was used directly for the next step.

### Step 4 Preparation of (4R)-7,7-dimethyl-4-(1-(methylsulfonyl)spiro[indoline-3,4'-piperidine]-1'-carbonyl)-6,9-dioxo-1-phenyl-2,10-dioxa-5,8-diazadodecane-11-ylisobutyrate (1e)

The compound **1d** (60 mg, 0.11 mmol) and Ibutamoren were dissolved in dichloromethane (3 mL), to above mixture was dropwise slowly added sodium hydroxide (22 mg, 0.22 mmol) dissolved in 5 mL of water . And the reaction mixture was stirred at 25°C for 2 h. The residue obtained by concentrating the organic layer was purified by preparative chromatography (acetonitrile/water) to give a compound **1e** (58 mg, white solid), and the yield was 72%.

MS m/z(ESI):687.0[M+1]⁺.

¹HNMR(400MHz, MeOD) δ 7.79-7.59(m, 1H), 7.42-7.26(m, 6H), 7.25-7.16(m, 1H), 6.99-6.90(m, 1H), 6.81-6.66(m, 1H), 5.18-5.11(m, 1H), 4.59-4.49(m, 2H), 4.18-3.97(m, 1H), 3.99-3.84(m, 2H), 3.81-3.63(m, 2H), 3.26-3.16(m, 2H), 2.96(d, J=6.4Hz, 3H), 2.87-2.8 (m, 1H), 2.52-2.50(m, 1H), 2.06-1.55(m, 4H), 1.51-1.31(m, 9H), 1.11-1.02(m, 6H).

### Step 5

The compound 1e prepared above was subjected to chiral resolution to give the compound represented by Formula (1), separation conditions: chromatographic column: Daicel CHIRALPAK IC_3, 3.0*150mm, 3µm, mobile phase: A/B: CO₂/MeOH=60/40, flow rate: 1.5 mL/min, and column temperature: 37°C.
t_{R}=1.582 min
MS m/z(ESI):687.0[M+1]⁺.
¹H NMR (300 MHz, dmso) δ 7.80 - 7.49 (m, 2H), 7.42 - 7.15 (m, 8H), 7.08 - 6.85 (m, 2H), 6.67 - 6.56 (m, 1H), 4.97 (d, *J =* 7.2 Hz, 1H), 4.44 (dd, *J =* 30.4, 12.1 Hz, 3H), 3.90 (d, *J* = 6.8 Hz, 3H), 3.72 - 3.45 (m, 2H), 3.15 (s, 1H), 3.04 (d, *J =* 2.2 Hz, 3H), 2.80 (s, 1H), 1.66 (s, 4H), 1.45 - 1.28 (m, 9H), 1.06 (d, *J =* 6.9 Hz, 6H).

The liquid chromatograph mass spectrometer LC-MS for determination was an Agilent 1200 Infinity Series mass spectrometer.

### Example 1 Amorphous substance

### 1. Analysis method

### 1.1 X-ray powder diffraction (XRPD)

A sample was subjected to crystal form analysis by an X-powder diffractometer. The 2θ scan angle of the sample was 3° to 42°, the scan step was 0.02°, and the scan time was 0.2 s per step. The tube voltage and current were 40 kV and 40 mA, respectively. When preparing the sample, an appropriate amount of sample was placed on a sample tray and flatted with a tool such as a spoon or glass sheet to make the surface smooth and flat.

### 1.2 Thermogravimetric analysis (TGA)

The sample was analyzed through TA Instruments TGA Discovery 550. The sample was placed in an aluminum tray with the tare removed, and was automatically weighted by the system, and then the sample was heated from room temperature to a specified temperature at a rate of 10 °C/min under nitrogen protection.

### 1.3 Differential scanning calorimetry (DSC)

The sample was analyzed through TA Instruments Discovery DSC 25. The weighed sample was placed in the sample tray and heated from 25 °C to the specified temperature at a rate of 10 °C/min under nitrogen protection (50 ml/min).

### 1.4 Dynamic vapor sorption analysis (DVS)

The sample was analyzed through Intrinsic DVS (System Measurement System UK). The amount of test sample was about 20-30 mg. The temperature of a test room was controlled between 25±1°C, the relative humidity increased from 0% to 90% and then decreased to 0% at a rate of 10%/h, and mass data was recorded every 20 s.

### 1.5 Scanning electron microscope (SEM)

The sample was analyzed through Phenom pure+. The sample was sprayed with melt and then placed in an instrument for testing. Different magnifications were adjusted to acquire the crystal habit of the sample.

### 1.6 Polarized light microscopy (PLM)

The sample was analyzed through the PLM, and different magnifications were adjusted to obtain the morphology and microstructure of crystals.

### 1.7 Nuclear magnetic resonance (¹H NMR) analysis

The sample was analyzed through a Varian Inova 500MHz NMR analyzer.

### 1.8 Liquid phase method (HPLC)

### Chromatographic conditions:

Chromatographic column: C18 column;
Running time: 60 min;
UV detector: 210 nm;
Flow rate: 1.0 mL/min;
Injection volume: 5 µL.

### 2. Preparation method

10 g of raw materials of the compound represented by Formula (1) were weighted and placed into a 100 ml glass bottle, an appropriate amount of DMSO was added at room temperature to completely dissolve and clarify, the dissolved clear solution was dropwise added to 100 ml of water, and stirred at room temperature to precipitate a solid, and then suction filtration was performed to obtain the solid.

### 3. Characterization of structural properties

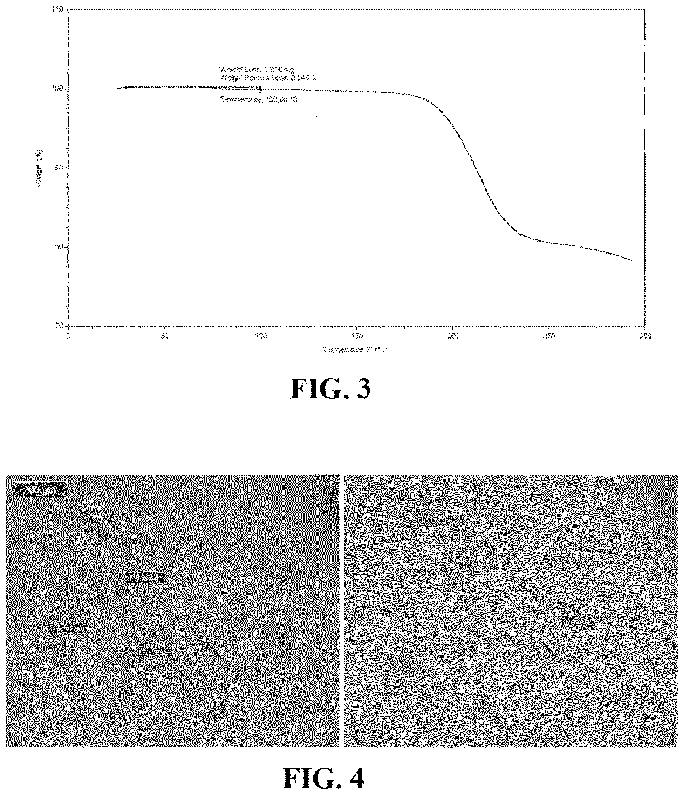

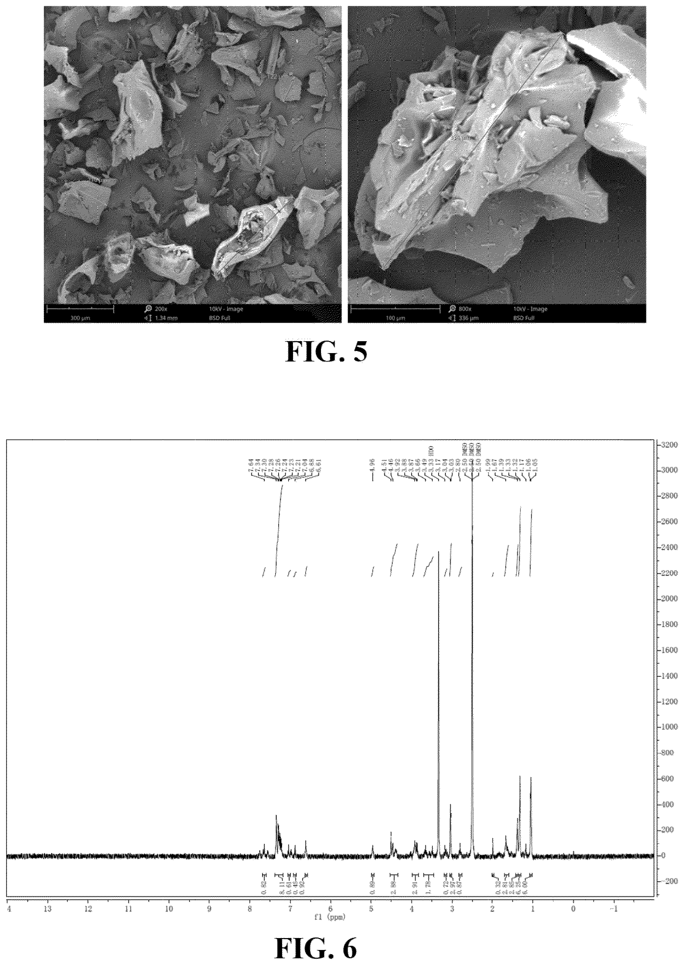

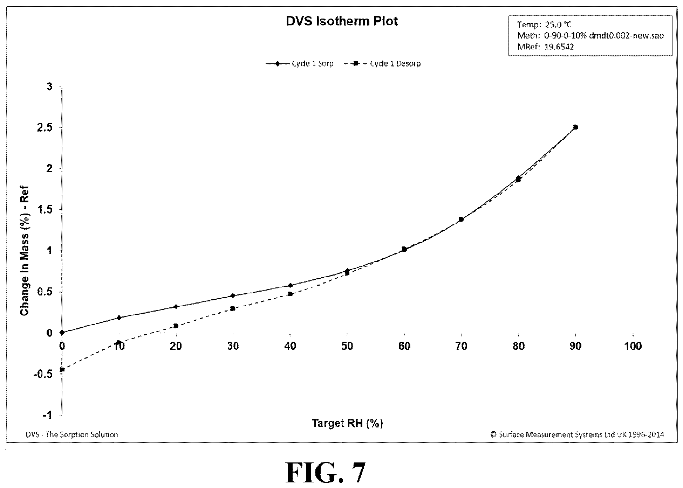

The obtained solid was detected, and the XRPD results (FIG. 1) showed that the obtained solid was the amorphous substance of the compound represented by Formula (1), and its ¹H NMR pattern was as represented by FIG. 6. A DSC curve (FIG. 2) showed that the glass-transition temperature of the amorphous substance was about 52.69°C, and a TGA curve (FIG. 3) showed that the amorphous substance had a weight loss of about 0.248% before 100°C. PLM (FIG. 4) and SEM (FIG. 5) showed that the amorphous substance was a massive amorphous body. DVS (FIG. 7) results showed that the amorphous substance was slightly hygroscopic at 80% humidity, and the morphology of the solid did not change before and after DVS test (FIG. 8).

### 4. Solubility test

About 10 mg of amorphous substance was weighted and placed into an 8 mL glass bottle, and a solvent was gradually added at room temperature. 5µL of solvent was added each time until all the solids were dissolved, and if 8 mL of solvent was added and the solids were not dissolved, the solvent addition was stopped. The specific experimental results are shown in Table 1.

**Table 1. Test results of solubility of amorphous substance of compound represented by Formula (1) (room temperature)**

| No. | Solvent | Volume (mL) | Amount of API (mg) | Phenomenon | Roughly measured solubility (mg/mL) |
|---|---|---|---|---|---|
| 1 | Methanol | 0.015 | 10.84 | Clear | >725 |
| 2 | Ethanol | 0.02 | 9.58 | Clear | >480 |
| 3 | Isopropanol | 0.02 | 8.64 | Clear | >432 |
| 4 | N-propanol | 0.015 | 6.90 | Clear | >460 |
| 5 | Tetrahydrofuran | 0.015 | 8.29 | Clear | >553 |
| 6 | Acetonitrile | 0.015 | 8.81 | Clear | >587 |
| 7 | Methyl tert-butyl ether | 0.09 | 6.14 | Clear | >70 |
| 8 | Acetone | 0.015 | 7.75 | Clear | >520 |
| 9 | Water | 8 | 10.43 | Turbid | <1.3 |
| 10 | Ethyl acetate | 0.015 | 8.96 | Clear | >600 |
| 11 | Isopropyl acetate | 0.015 | 9.64 | Clear | >643 |
| 12 | Toluene | 0.015 | 7.09 | Clear | >473 |
| 13 | Ethyl formate | 0.02 | 8.07 | Clear | >405 |
| 14 | N-heptane | 8 | 11.02 | Turbid | <1.3 |
| 15 | Dichloromethane | 0.015 | 11.97 | Clear | >798 |
| 16 | 1,4-Dioxane | 0.01 | 7.60 | Clear | >760 |
| 17 | Cyclohexane | 8 | 9.27 | Turbid | <1.2 |
| 18 | Methyl isobutyl ketone | 0.02 | 7.99 | Clear | >400 |
| 19 | Butanone | 0.01 | 6.99 | Clear | >700 |
| 20 | N-hexane | 4 | 5.85 | Turbid | <1.2 |
| 21 | Anisole | 0.035 | 18.18 | Clear | >510 |
| 22 | Dimethyl sulfoxide | 0.015 | 6.79 | Clear | >450 |

As shown in the above table, the amorphous substance of the compound represented by Formula (1) have low solubility in water, n-heptane, n-hexane and cyclohexane, and have high solubility in other solvents.

### Example 2 Crystal form

### 1. Analysis method

### 1.1 X-ray powder diffraction (XRPD)

An XRPD spectrum was acquired from a PANalytical X-ray powder diffraction analyzer with the following scanning parameters:

| Parameters | XRPD (Reflection mode) |
|---|---|
| Model | X'Pert3 |
| X-ray | Cu, kα, Kα1 (Å):1.540598; Kα2 (Å):1.544426; Kα2/Kα1 Intensity ratio: 0.50 |
| X-ray tube setting | 45 kV, 40 mA |
| Divergence slit | Fixed 1/8° |
| Scan mode | Continuous |
| Scan range (°2Theta) | 3-40 |
| Scan time per step (s) | 46.7 |
| Scan step (°2Theta) | 0.0263 |
| Test Time (min) | 5 |

### 1.2 Thermogravimetric analysis (TGA) and differential scanning calorimetry (DSC)

TGA and DSC plots were acquired from the TA Discovery TGA 5500 thermogravimetric analyzer and the TA Discovery DSC 2500 differential scanning calorimeter, respectively, with the following test parameters:

| Parameters | TGA | DSC |
|---|---|---|
| Method | Linear temperature increase | Linear temperature increase |
| Sample tray | Aluminum tray, opened | Aluminum tray, covered |
| Temperature range | Room temperature-set end temperature | 25°C-set end temperature |
| Scan rate (º C/min) | 10 | 10 |
| Protective gas | Nitrogen | Nitrogen |

### 1.3 Dynamic vapor sorption (DVS)

The DVS curve was acquired from DVS Intrinsic through an SMS (Surface Measurement Systems). The relative humidity at 25°C was calibrated according to deliquescent points of LiCl, Mg(NO₃)₂ and KCl. The DVS test parameters are as follows:

| Parameters | Set value |
|---|---|
| Temperature | 25°C |
| Sample amount | 10-20 mg |
| Protective gas and flow rate | N₂, 200 mL/min |
| dm/dt | 0.002%/min |
| Minimum dm/dt equilibrium time | 10 min |
| Maximum equilibrium time | 180 min |
| RH range | 0%RH ~ 95%RH ~ 0%RH |
| RH gradient | 10%RH (0%RH-90%RH & 90%RH-0%RH) |
| | 5%RH (90%RH-95%RH & 95%RH-90%RH) |

### 1.4 Solution nuclear magnetic resonance (solution NMR)

A solution NMR pattern was acquired from a Bruker 400M NMR instrument (Jiangsu Jitri Optoelectronics Testing Center Co., Ltd.), and DMSO-d6 was used as an NMR test solvent.

### 2. Preparation method

About 15 mg of raw material solid of the compound represented by Formula (1) was placed in a glass bottle, and an appropriate amount of mixed solvent of acetone and water was added to the glass bottle and the mixture was suspended and stirred at room temperature. And then the mixture was subjected to suction filtration to give the solid.

### 3. Characterization of structural properties

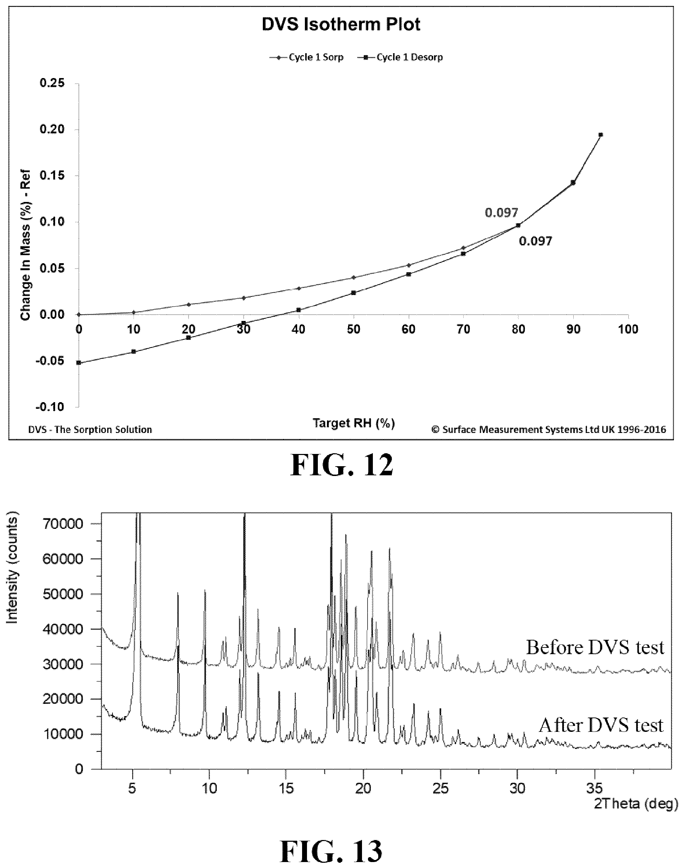

The solid sample prepared through the above method was subjected to XRPD test (as shown in FIG. 9), and a crystal form was shown in the result. The TGA curve (FIG. 10) showed that the crystal had a 1.1% weight loss when being heated to 150°C, and the DSC curve (FIG. 11) showed an endothermic peak was observed at 130.6°C (peak temperature). Based on the small TGA weight loss of the crystal form and the absence of DSC signal before 100°C, it was speculated that the crystal form was an anhydrous crystal form. In addition, the crystal form was subjected to DVS test at a constant temperature of 25°C to evaluate the hygroscopicity. The DVS results represented by FIG. 12 showed the moisture adsorption of the crystal form at 25°C/80%RH was 0.10%, indicating that the crystal form almost had no hygroscopicity. The XRPD results (FIG. 13) showed that the crystal form did not vary before and after the DVS test.

### 3.1 X-ray powder diffraction (XRPD)

A sample was subjected to crystal form analysis by an X-powder diffractometer. When preparing the sample, an appropriate amount of sample was placed on a sample tray and flatted with a tool such as a spoon or glass sheet to make the surface smooth and flat. The results are shown in Table 2 and FIG. 9.

**Table 2. XRPD peak data of crystal form of compound represented by Formula (1)**

| **Peak position [°2θ]** | **Relative intensity [%]** |
|---|---|
| 5.4352 | 100.00 |
| 7.9648 | 6.23 |
| 9.7192 | 7.21 |
| 10.8697 | 2.50 |
| 11.0751 | 2.77 |
| 11.9715 | 4.68 |
| 12.2778 | 17.64 |
| 13.1603 | 5.60 |
| 14.5028 | 3.95 |
| 15.2509 | 1.00 |
| 15.5493 | 3.90 |
| 16.1982 | 1.12 |
| 16.5168 | 1.17 |
| 17.0868 | 0.34 |
| 17.6924 | 6.00 |
| 17.9106 | 18.21 |
| 18.1650 | 6.91 |
| 18.5393 | 10.63 |
| 18.8711 | 12.67 |
| 19.5143 | 5.97 |
| 20.3083 | 7.98 |
| 20.5388 | 10.69 |
| 20.8176 | 4.48 |
| 21.6912 | 12.00 |
| 21.8594 | 8.41 |
| 22.3867 | 1.34 |
| 22.5986 | 1.87 |
| 23.2500 | 3.47 |
| 24.1879 | 3.00 |
| 24.6722 | 0.81 |
| 24.9742 | 3.77 |
| 25.8055 | 0.70 |
| 26.1376 | 1.48 |
| 27.4319 | 0.87 |
| 28.4451 | 1.00 |
| 29.3745 | 1.32 |
| 29.6008 | 1.13 |
| 30.0031 | 0.74 |
| 30.4474 | 1.18 |
| 31.2540 | 0.68 |
| 31.8517 | 0.95 |
| 32.2825 | 0.74 |
| 33.3883 | 0.42 |
| 34.6974 | 0.29 |
| 35.2546 | 0.54 |
| 36.9963 | 0.17 |
| 38.1733 | 0.35 |
| 38.7760 | 0.28 |
| 39.2228 | 0.56 |

### 4. Solubility test

About 5 mg of the crystal form sample was weighed and placed in an 8 mL glass bottle, and solvent was gradually added at room temperature. 10 uL of solvent was added each time until all solids were dissolved, and if 5 mL of solvent was added and the solids were not dissolved, the solvent addition was stopped. The specific experimental results are shown in Table 3.

**Table 3. Test results of solubility of crystal form of compound represented by Formula (1) (room temperature)**

| No. | Solvent | Solubility (mg/mL) |
|---|---|---|
| 1 | Ethanol | ~ 20.9 |
| 2 | Tetrahydrofuran | ~ 232.5 |
| 3 | Acetonitrile | ~ 128 |
| 4 | Methyl tert-butyl ether | ~ 0.7 |
| 5 | Acetone | ~ 117 |
| 6 | Water | <0.7 |
| 7 | Ethyl acetate | ~ 132.8 |
| 8 | Toluene | ~ 13.2 |
| 9 | N-heptane | <0.6 |
| 10 | Dichloromethane | ~ 251 |

As shown in Table 3, the crystal form has low solubility in water, n-heptane and methyl tert-butyl ether, and has high solubility in other solvents.

### Example 3 Stability test 1

A certain amount of crystals and amorphous raw materials were weighed and placed in liquid phase vials, and a total of 5 parts were prepared. The 5 parts were respectively placed under 80°C (opened), 25°C/60% RH (opened), and 40°C/75% RH (opened) and in a stable lighting chamber (closed, 5000±500 Lx), and the stability under lighting for 10 days, 80°C 1 day, and the chemical stability under 25 °C and 60% RH and under 40°C and 75% RH for 1 week were determined by liquid phase, and the XRPD of the solid was determined. The specific results are shown in Table 4. The results show that the amorphous substance and the crystal form of the compound represented by Formula (1) have better physical and chemical stability under most conditions, but the stability of the crystal form is significantly better than that of the amorphous substance under lighting conditions.

**Table 4. Test results of stability of amorphous substance and crystal form of compound represented by Formula (1)**

| Conditions | | Amorphous substance | | Crystal form | |
|---|---|---|---|---|---|
| | | Purity (%) | Degradation (%) | Purity (%) | Degradation (%) |
| Initial | | 99.28 | N/A | 99.71 | N/A |
| 80°C | 1 Day | 99.10 | 0.18 | 99.59 | 0.12 |
| Lighting | 10 Days | 98.26 | 1.02 | 99.36 | 0.35 |
| 25°C, 60%RH | 1 week | 99.27 | 0.01 | 99.69 | 0.02 |
| 40°C, 75%RH | 1 week | 99.25 | 0.03 | 99.61 | 0.1 |

Accelerated stability test:
The crystal sample was weighed, 4 g per part, and it was observed in a constant-temperature and constant-humidity chamber with a temperature of 40±2°C and a relative humidity of 75±5%. The sample was taken at 0th, 1st and 3rd month for analysis, the stability of the samples was determined by liquid phase, and the crystal form was determined by XRPD. The specific results are shown in Table 5. The results show that the crystal form of the compound represented by Formula (1) still has good stability under acceleration conditions.

**Table 5. Results of the accelerated stability test of crystal form of compound represented by Formula (1)**

| Test items | Initial | 1 month | 3 months |
|---|---|---|---|
| Crystal form | Crystal form shown in FIG. 9 | Consistent with the pattern in FIG. 9, the crystal form did not vary | Consistent with the pattern in FIG. 9, the crystal form did not vary |
| Total impurities (%) | 0.07 | 0.07 | 0.05 |

### Example 4 Stability test 2

Packaging requirements: it is needed to simulate commercially available packaging for the stability sample, the two inner layers are inner flat-bottom bags made of pharmaceutical polyethylene, the first layer of inner bag is tied and sealed, the second layer of inner bag is subjected to heat sealing, an aluminum foil bag at an outer layer is subjected to heat sealing, a package of desiccant is placed between the aluminum foil bag and the second layer of inner bag, and the package is placed in a fiber can.

### 1.1 Accelerated stability test

The crystal form of the compound represented by Formula (1) was packaged according to the above packaging requirements, the sample was conventionally sub-packaged into 5 parts, 10 g per part, and it was observed in a constant-temperature and constant-humidity chamber with a temperature of 40±2°C and a relative humidity of 75±5%. The sample was taken in the 0th, 1st, 2nd, 3rd and 6th months. The test items, test methods and technical requirements are detailed in Table 6, and the test results are compared with those in the 0th month.

### 1.2 Long-term stability test

The crystal form of the compound represented by Formula (1) was packaged according to the above packaging requirements, the sample was conventionally sub-packaged into 10 parts, 10 g per part, and it was observed in the constant-temperature and constant-humidity chamber with a temperature of 30±2°C and a relative humidity of 65±5%. The sample was taken in the 0th, 3rd and 6th months. The test items, test methods and technical requirements are detailed in Table 7, and the test results are compared with those in the 0th month.

**Table 6: Acceleration test (40±2°C/75±5%RH)**

| Name of indexes | | 0th month | 1st month | 2nd month | 3rd month | 6th month |
|---|---|---|---|---|---|---|
| Characters | | White powder | White powder | White powder | White powder | White powder |
| Identification | Infrared spectroscopy | Consistent with the pattern of a reference product | Consistent with the pattern of a reference product | Consistent with the pattern of a reference product | Consistent with the pattern of a reference product | Consistent with the pattern of a reference product |
| | High-performance liquid chromatography | Consistent with the retention time of the reference product at main peak | Consistent with the retention time of the reference product at main peak | Consistent with the retention time of the reference product at main peak | Consistent with the retention time of the reference product at main peak | Consistent with the retention time of the reference product at main peak |
| Moisture | | 0.2% | 0.1% | 0.2% | 0.1% | 0.1% |
| Related substances /HPLC | Impurity 1 | ND | ND | ND | ND | ND |
| | Impurity 2 | ND | ND | ND | ND | ND |
| | Impurity 3 | <0.05% | <0.05% | <0.05% | <0.05% | <0.05% |
| | Impurity 4 | ND | ND | ND | ND | ND |
| | Other impurities | 0.05% | 0.05% | 0.05% | <0.05% | <0.05% |
| | Total impurities | 0.05% | 0.05% | 0.05% | <0.05% | <0.05% |
| Isomer/HP LC | Isomer 1 | 0.05% | 0.04% | 0.04% | 0.04% | 0.04% |
| | Isomer 2 | ND | ND | ND | ND | ND |
| | Isomer 3 | ND | ND | ND | ND | ND |
| | Chiral purity | 100.0% | 100.0% | 100.0% | 100.0% | 100.0% |
| Content (anhydrous and insoluble residue)/HPLC | | 99.4% | 99.0% | 98.9% | 98.9% | 99.5% |
| Crystal form/XRPD | | Consistent with the pattern of crystal form | / | / | Consistent with the pattern of crystal form | Consistent with the pattern of crystal form |

| Name of indexes | | 0th month | 1st month | 2nd month | 3rd month | 6th month |
|---|---|---|---|---|---|---|
| Conclusion | | Qualified | Qualified | Qualified | Qualified | Qualified |

| | | | | | | |
|---|---|---|---|---|---|---|
| Note: The data of the 0th month was obtained based on the release results; ND refer to not detected. | | | | | | |

**Table 7: Long-term test (30±2°C/65±5%RH)**

| **Name of indexes** | | 0th month | 3rd month | 6th month |
|---|---|---|---|---|
| Characters | | White powder | White powder | White powder |
| Identification | Infrared spectroscopy | Consistent with the pattern of a reference product | Consistent with the pattern of a reference product | Consistent with the pattern of a reference product |
| | High-performance liquid chromatography | Consistent with the retention time of the reference product at main peak | Consistent with the retention time of the reference product at main peak | Consistent with the retention time of the reference product at main peak |
| Moisture | | 0.2% | 0.1% | 0.1% |
| Related substance/HPLC | Impurity 1 | ND | ND | ND |
| | Impurity 2 | ND | ND | ND |
| | Impurity 3 | <0.05% | <0.05% | <0.05% |
| | Impurity 4 | ND | ND | ND |
| | Other impurities | 0.05% | 0.05% | <0.05% |
| | Total impurities | 0.05% | 0.05% | <0.05% |
| Isomer/HPLC | Isomer 1 | 0.05% | 0.06% | 0.03% |
| | Isomer 2 | ND | ND | ND |
| | Isomer 3 | ND | ND | ND |
| | Chiral purity | 100.0% | 99.9% | 100.0% |
| Content (anhydrous and insoluble residue)/HPLC | | 99.4% | 99.9% | 99.6% |
| Crystal form/XRPD | | Consistent with the pattern of crystal form | / | / |
| Conclusion | | Qualified | Qualified | Qualified |

| | | | | |
|---|---|---|---|---|
| Note: The data of the 0th month was obtained based on the release results; ND refer to not detected. | | | | |

The results of the accelerated stability test showed that there was no significant change in the detection results at the 6th month compared with the 0th month, and there was no new impurity peak greater than 0.10%, which indicates that the crystal form of the compound represented by Formula (1) was stable under the accelerated condition at 6 months according to the above existing packaging methods.

The results of the long-term stability test showed that there was no significant change in the detection results at the 6th month compared with the 0th month, and there was no new impurity peak greater than 0.10%, which indicates that the crystal form of the compound represented by Formula (1) was stable under the long-term condition at 6 months according to the above existing packaging methods.

### Example 4

Expanded production was carried out according to the method in the Example 2, and 10 kg of solid sample could be prepared, and the crystal form shown in FIG. 9 was shown in the XRPD test result.

### Example 5 Biological activity test

### Test example 1: Determination of activity of compound of the present invention on human GHSR

This method is used for determining the agonistic activity effect of the compound in the present invention on human GHSR protein expressed in human GHSR/CHO stable transformant cells.

### 1. Test materials and instruments

### 1. Culture medium

F12 (Gibco, Cat#11765-047);
FBS (Corning, Cat# 35-076-CV);
Geneticin (Invitrogen, Cat# 10131);
Penicillin/Streptomycin (Invitrogen, Cat# 15140).

### 2. Reagents

Fluo-4 Direct (Invitrogen, Cat# F10471);
HBSS (Gibco, Cat#14025076);
HEPES (Gibco, Cat#15630080);
Bonine Serum Albumin (Sgima, Cat#B2064-100G).

### 3. Instrument consumables

384 well Poly-D-Lysine protein coating plate (Greiner, Cat#781946);
FLIPR (Molecular Devices);
Vi-cell XR Cell Viability Analyzer (Beckman Coulter);
Incubator (Thermo).

### 2. Experimental steps

Gradient preparation of compound: Ghrelin and the compound in the present invention were diluted 5 times to prepare 10 concentration gradients, and then transferred to a compound plate, 900 nL was placed in each well.
Preparation of buffer solution: HBSS (1X): HEPES (1M) = 49:1, 0.5% BSA added.

Human GHSR/CHO containing stable transformant cells were inoculated in a 384-well plate, a cell plate was taken out after overnight, the culture medium was removed, and 20 µL of buffer solution was slowly added in each well, then 20 µL of 2X Fluo-4 Direct^{™} No-wash Loading Buffer was added in each well. The cell plate was incubated in an incubator containing 5% of CO₂ at 37°C for 50 min, and the cell plate was taken out and placed at room temperature for 10 min. 30 uL of buffer solution was added to each well in the compound plate; an additional buffer solution plate was prepared, and 30 µL of buffer solution was added to each well. For test of agonistic effect of compound: an FLIPR instrument was used, and software was operated. 10 µL of buffer solution was transferred to the cell plate, the fluorescence signal values were read. 10 uL of compound was transferred to the cell plate, the fluorescence signal values were read. The maximum-minimum values from the 91st signal point to the 230th signal point were calculated through the FLIPR program. The EC₅₀ value of the compound could be calculated by the software using the fluorescence value corresponding to the different concentrations.

### 3. Experimental results:

The agonistic activity of the compound 1e on human GHSR is determined by the above experiments, and the determined EC₅₀ value is 14.6 nM, which indicates that compound 1e has good agonistic activity to human GHSR. Therefore, it is confirmed that the compound represented by Formula (1) has good agonistic activity to human GHSR.

### Test example 2: Caco-2 cell transport experiment

A transport buffer solution in the study was HBSS containing 10.0 mM of HEPSS, and had pH of 7.40±0.05. The compound to be tested was subjected to bidirectional test at 2.00 µM, and the final concentration of DMSO was required to be less than 1%. In a CO₂ incubator at 37±1°C, the cell plate was stood and incubated for 2 h under CO₂ at 5% humidity and saturated humidity. After mixing all samples with acetonitrile containing internal standard, the mixture was centrifuged at 3200 xg for 10 min. The compound was tested, 100 µL of supernatant was diluted with 100 µL of ultrapure water for LC-MS/MS analysis. The concentration of the test compound and a control compound (Digoxin as a model verification compound and ibutamoren as a positive control) in a donor solution and a receiver solution was quantified by an LC-MS/MS method using the peak area ratio of the analyte/internal standard. After transport determination, a lucifer yellow exclusion determination was carried out to determine the integrity of the Caco-2 cell monolayer.

**Table 8 Caco-2 cell transport experiment of compound 1e in example**

| **Sample** | **Mean Pₐₚₚ (10⁻⁶ cm/s)** | | **Efflux Ratio** |
|---|---|---|---|
| | **A to B** | **B to A** | |
| Digoxin | 0.0229 | 12.0 | 523 |
| ibutamoren | 0.0281 | 19.5 | 694 |
| Compound 1e | 0.0518 | 8.80 | 170 |

The above data show that the model is successfully constructed, and the cell permeability of compound 1e is significantly better than that of the reference compound ibutamoren.

### Test example 3: Comparison of in-vivo metabolism experiments of compound 1e in rats

The compound 1e was subjected to prodrug design, and the amount of active ingredient ibutamoren was tested by in-vivo metabolism experiments in rats, so as to evaluate the advantages and disadvantages of a candidate and a positive control ibutamoren.

### Experimental operations:

6 animals were fasted for the first time two days before drug administration, and after fasting for at least 12 h, they were uniformly fed; the animals were fasted for the second time the day before drug administration, fasting was performed for at least 12 h, and feeding was resumed 4 h after drug administration. Each fasting did not exceed 20 h. The animals could drink water freely during this period. Within two days before drug administration, the same person in charge would touch and grab the animals for adaptive training, and the adaptive training was performed at least 1 time a day.

Before the first drug administration, the animals were divided into 2 groups by body weight. There were three animals in each group, the first group of animals were administrated with drug liquid of a compound 5 by a single gavage (preparation method: medium chain triglycerides/polyethylene glycol 1000 vitamin E succinate/ethanol/propylene glycol/water=6/2/1/1/90, 1 mg/mL); the second group of animals were administrated with drug liquid of ibutamoren (water, 1 mg/mL) by a single gavage. The volume for drug administration was 3 mL/kg. The animals were weighed before drug administration, and the volume for drug administration was calculated based on the body weight.

Sample collection time: before drug administration (about -0.25 h) and 0.083, 0.25, 0.5, 1, 1.5, 2, 3, 5, 7, 10 h after drug administration; at each specified time point, the animals were briefly anesthetized with isoflurane, whole blood samples (approximately 0.23 mL per group) were collected by jugular venous puncture, and 50 µL of whole blood samples were quantitatively taken, added to an EP tube filled with 50 µL of pre-cooled 1 mM PMSF methanol solution, and vortexed for about 3s; then 250 µL of precipitant containing internal standard was immediately added, vortexed for about 5 s, and centrifuged for 15 min to obtain supernatant for LC-MS/MS analysis.

**Table 9: Experimental data of in-vivo metabolism of drugs in rats**

| **Group** | **T_{1/2} (hr)** | **Tₘₐₓ (hr)** | **Cₘₐₓ (ng/mL)** | **AUCₗₐₛₜ (hr*ng/mL)** | **AUCINF-obs (hr*ng/mL)** |
|---|---|---|---|---|---|
| **Compound 1e** | 2.43±0.13 | 0.44±0.49 | 18.50±13.60 | 21.61±9.52 | 23.31±10.81 |
| ibutamoren | 9.61±12.07 | 1.06±1.68 | 3.49±2.81 | 6.97±3.27 | 14.28±11.03 |

The above data show that the compound 1e in the present invention has Cₘₐₓ and AUC significantly better than that of the positive control ibutamoren in the drug metabolism experiment of intragastric administration in rats, and has better drug formation performance. It is confirmed that the compound represented by Formula (1) has better drug formation performance.

Through the above-mentioned experiments, the crystal form and amorphous substance of the prepared compound in the present invention have high purity, have good stability under high temperature and high humidity, and are conducive to the functioning of the drug; and for process optimization, the production process stability, repeatability and controllability are met, and industrial production can be performed.

The embodiments of the present invention are described above. However, the present invention is not limited to the above embodiments. Any modifications, equivalent substitutions, improvements, etc., made within the spirit and principles of the present invention shall be comprised in the protection scope of the present invention.

## Claims

1. A crystal form of a compound represented by Formula (1), wherein an X-ray powder diffraction pattern of the crystal form comprises characteristic peaks at 5.43±0.20°, 12.28±0.20° and 17.91±0.20° 2θ, as determined by using Cu-Kα radiation;

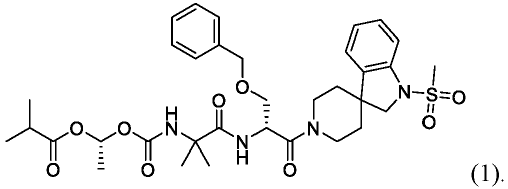

2. The crystal form according to claim 1, wherein the X-ray powder diffraction pattern of the crystal form comprises characteristic peaks at 5.43±0.20°, 12.28±0.20°, 17.91±0.20°, 18.54±0.20°, 18.87±0.20°, 20.54±0.20° and 21.69±0.20° 2θ, as determined by using Cu-Kα radiation.

3. The crystal form according to claim 1 or 2, wherein the X-ray powder diffraction pattern of the crystal form comprises characteristic peaks at 5.43±0.20°, 7.96±0.20°, 9.72±0.20°, 12.28±0.20°, 13.16±0.20°, 17.69±0.20°, 17.91±0.20°, 18.16±0.20°, 18.54±0.20°, 18.87±0.20°, 19.51±0.20°, 20.31±0.20°, 20.54±0.20°, 21.69±0.20° and 21.86±0.20° 2θ, as determined by using Cu-Kα radiation.

4. The crystal form according to any one of claims 1 to 3, wherein the crystal form has an XRPD spectrum substantially represented by FIG. 9; and preferably, the crystal form is an anhydrous substance.

5. A preparation method of the crystal form according to any one of claims 1 to 4, comprising the following steps: dissolving the compound represented by Formula (1) in a solvent A, and cooling the solution to precipitate a solid so as to obtain the crystal form, wherein the solvent A is selected from a group consisting of methanol, ethanol, isopropanol, tert-butanol, n-butanol, acetone, tetrahydrofuran, methyltetrahydrofuran, ethyl formate, ethyl acetate, isopropyl acetate, n-hexane, n-heptane, cyclohexane, methyl tert-butyl ether, toluene, dichloromethane, chloroform, DMSO, water, acetonitrile, isopropyl ether, and combinations of two or more solvents thereof.

6. An amorphous substance of a compound represented by Formula (1), wherein an XRPD spectrum of the amorphous substance does not have obvious diffraction peak; and 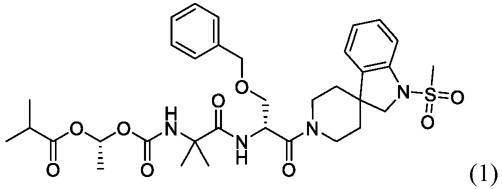 preferably, the amorphous substance has an XRPD spectrum substantially represented by FIG. 1.

7. A preparation method of the amorphous substance according to claim 6, comprising the following steps: dissolving the compound represented by Formula (1) in a solvent B, adding the obtained clear solution to a solvent C, and stirring to precipitate a solid so as to obtain the amorphous substance, wherein
the solvent B is a good solvent for the compound represented by Formula (1), and the solvent C is a poor solvent for the compound represented by Formula (1).

8. A pharmaceutical composition, comprising the crystal form according to any one of claims 1 to 4 and/or the amorphous substance according to claim 6;
preferably, the pharmaceutical composition further comprises pharmaceutically acceptable excipients;
preferably, the pharmaceutical composition further comprises a second active ingredient, besides the crystal form and/or the amorphous substance, for example, the second active ingredient is a growth development-related drug; and
preferably, the pharmaceutical composition is a pharmaceutical formulation, for example, a GHSR agonist.

9. Use of the crystal form according to any one of claims 1 to 4, the amorphous substance according to claim 6 and/or the pharmaceutical composition according to claim 8 for the manufacture of a medicament for diagnosing, preventing and/or treating growth hormone deficiency or growth hormone-dependent diseases (or disorders).

10. A method for diagnosing, preventing and/or treating growth hormone deficiency or growth hormone-dependent diseases (or disorders), comprising: administering a therapeutically effective amount of the crystal form according to any one of claims 1 to 4, the amorphous substance according to claim 6 or the pharmaceutical composition according to claim 8 to patients.
